# EUROPEAN PATENT SPECIFICATION

(11) **EP 2 342 337 B1**
(45) Date of publication and mention of the grant of the patent: **27.05.2015**
(21) Application number: 09788156.9
(22) Date of filing: 04.09.2009
(51) Int. Cl.: C12N 15/10, C12Q 1/68

(54) **METHOD FOR DIAGNOSTIC MARKER DEVELOPMENT**
VERFAHREN ZUR ENTWICKLUNG DIAGNOSTISCHER MARKER
PROCÉDÉ POUR LE DÉVELOPPEMENT DE MARQUEURS DIAGNOSTIQUES

(30) Priority: 11.09.2008 US 96054 P
(43) Date of publication of application: 13.07.2011
(73) Proprietor: KEYGENE N.V., 6700 AE Wageningen (NL)
(72) Inventor: ROUPPE VAN DER VOORT, Jeroen Nicolaas Albert Maria, NL-6703 BR Wageningen (NL); SØRENSEN, Anker Preben, NL-6871 HZ Renkum (NL)
(74) Representative: EP&C
(86) International application number: PCT/NL2009/000172
(87) International publication number: WO 2010/030171

(56) References cited:
- WO-A2-2004/033708
- XU YUNBI ET AL: "Marker-assisted selection in plant breeding: From publications to practice" THE PLANT GENOME, CROP SCIENCE SOCIETY OF AMERICA, MADISON, WI, US, vol. 48, no. 2, 1 March 2008 (2008-03-01), pages 391-407, XP009110684 ISSN: 0011-183X
- YI JI-CAI ET AL: "Genetic analysis and molecular mapping of a rolling leaf mutation gene in rice" JOURNAL OF INTEGRATIVE PLANT BIOLOGY, vol. 49, no. 12, December 2007 (2007-12), pages 1746-1753, XP002565183 ISSN: 1672-9072
- ZHU TONG ET AL: "Engineering herbicide-resistant maize using chimeric RNA/DNA oligonucleotides" NATURE BIOTECHNOLOGY, vol. 18, no. 5, May 2000 (2000-05), pages 555-558, XP002565184 ISSN: 1087-0156
- S. PEÑUELA ET AL: "Targeted isolation, sequence analysis, and physical mapping of nonTIR NBS-LRR genes in soybean", TAG THEORETICAL AND APPLIED GENETICS, vol. 104, 1 February 2002 (2002-02-01), pages 261-272, XP055077609, ISSN: 0040-5752, DOI: 10.1007/s00122-001-0785-0

## Description

The present invention relates to a method for the development of a molecular marker in an organism, in particular a diagnostic marker. The method further relates to the introduction, in a germplasm of an organism, of a selectable marker and to the use of the marker in a breeding process of, for instance, a plant.

Since a decade, DNA marker technology is dramatically enhancing the efficiency of plant breeding by allowing selection on the basis of easy to assay markers instead of the often difficult to phenotype traits. However, the development of such diagnostic markers and the effectiveness of applying these markers is often a laborious and time consuming process. Firstly, the development of diagnostic markers follows a process starting with
1) mapping the genetic position of the gene(s) underlying the trait of interest,
2) identification of flanking markers,
3) fine mapping of the gene(s) by identification of tightly linked markers,
4) determination of the DNA marker sequences of the most linked markers,
5) determination of the sequence variation at the marker loci between the parent lines used to map the target gene,
6) development of simple PCR assays,
7) test of predictive value in the genetic background (germplasm) of the plant material where the diagnostic marker will be tested.

Germplasm, as used herein, is a term used to describe the genetic resources, or more precisely the DNA of an organism and collections of that material. Breeders use the term germplasm to indicate their collection of genetic material from which they can draw to create varieties.

Fortunately, recent developments have significantly speeded up steps 1 - 4 (Morgante and Salamini 2003 Curr. Opinion in Biotechnol 14: 214 - 219; Varshney et al. 2005 TiPS 10: 621 - 630), especially when dealing with monogenic traits. Steps 5 - 6 are relying on the occurrence of a stretch of unique DNA marker sequences around the sequence variants (SNPs) used to develop the diagnostic assay. Plant genomes are richly dispersed with repetitive sequences which obstructs significantly possibilities of the development of diagnostic marker assays. Especially for crops with large genome sizes, the identification of low copy DNA segments can be described as searching for a needle in a haystack. Finally, step 7 is very much depending on 1) the mating system of the crop and 2) the level of genetic variability in the crop genome. When a functional mutation occurs, this mutation will be on a haplotype of other pre-existing DNA polymorphisms. As the trait is transmitted through subsequent generations of random mating, many recombination events will occur which will render the original haplotype into small linkage blocks. This will cause the trait gene to be separated from most of the specific alleles of its original haplotype. The consequence of this is that only DNA polymorphisms with extremely tight linkage to the trait gene or even the causal polymorphism itself can be exploited to be converted into useful DNA markers. As an example in outbred random mating populations, recombinations and a high exchange rate of recombination events result in trait-containing regions that are (theoretically) extremely small. Hence, DNA markers that are perfectly associated with the genes will be very hard to find. As a contrary example, linkage blocks in inbred populations tend to be relatively large because selfing increases homozygosity, thereby limiting the number of heterozygotes that can be shuffled by recombination. However, for crops where the genetic basis for the cultivated species is very narrow, it will be difficult to identify any DNA polymorphisms as starting points for DNA marker development, despite the occurrence of relative large linkage blocks.

WO 2004/033708 discloses a method for introducing an alteration in a trait of an organism by TNE, wherein both the sequence of the trait and the sequence of a selectable marker is altered by TNE. Zhu (Nature Biotechnology (2000), Vol. 18, no 5, pages 555 - 558) uses chimeric RNA/DNA oligonucleotides to target endogenous genes to engineer maize plants resistant to imidazolinone herbicides.

Xu (Crop Science (2008), Vol 48, pages 391 - 407) and Yi (Journal of integrative plant biology (2007), Vol 4, issue 12, pages 1746 - 1753) both concern marker assisted selection of plants and marker identification, showing the use of SNP markers in breeding procedures.

The present inventors have set out to develop a method for the creation of selectable markers that do not suffer from the above problems.
In this application, a method is described to introduce unique, artificial and selectable markers at targeted regions instead of identifying and exploiting naturally occurring sequence variants. The rationale behind this method is that, based on sequence knowledge at potential marker loci, unique markers can be designed and introduced in the line(s) that harbor the trait of interest. Preferred for the applicability of such markers is that the novel introduced markers co-segregate consistently over many generations with the trait of interest. The present inventors have found a method to introduce markers within targeted regions in such a way that they are useful as markers with a broad predictive value.

The present inventors have discovered a strategy based on identifying and/or selecting a section of DNA that is closely linked to (i.e. with a pre-defined consistency co-segregating with) the trait or traits of interest and converting this section into a selectable molecular marker using a method for targeted mutagenesis, targeted nucleotide exchange (TNE), thereby overcoming the above difficulties. In particular, the present inventors have found a method for determining which alterations have to be made and to design the desired marker such that any genetic variability is reduced to a minimum and the quality of the marker, i.e. its predictive value for the associated trait is optimal within a germplasm, but that may also be useful in a different germplasm than from which the marker was originally developed.

The present invention thus relates to a method for the introduction of one or more (unique and selectable) molecular markers in a germplasm, which markers are closely linked to a trait of interest and which markers are located at a position in the genome that, although closely linked to the desired trait, did not contain adequate marker information. In this section of the DNA an artificial and unique polymorphism is created using TNE that subsequently can serve as a selectable molecular marker. The present invention also relates to the use of TNE for the creation of unique and selectable markers in an organism.

The invention is as defined in the claims.

Thus, in a first aspect the disclosure pertains to a method for introducing a unique and selectable SNP in a marker associated with a trait in a non-human organism comprising the steps of:
- selecting a trait of interest;
- determining a locus that is associated with the trait;
- determining the genetic map position of the locus;
- identifying a marker that is located within a genetic distance from the locus;
- providing a population of the organism wherein each member of the population contains the marker;
- determining the nucleotide sequence of the marker for each of the members of the population;
- aligning the nucleotide sequences of the markers;
- selecting at least one position in the sequence of the markers that contain the same nucleotide in all markers of the population;
- designing an oligonucleotide that is capable of hybridising to the marker sequence adjacent to both sides of the at least one position and wherein the oligonucleotide further contains a nucleotide (the marker nucleotide) at the at least one position that is different from the nucleotide at the at least one position in the marker;
- introduce the marker nucleotide in the DNA of the organism using targeted nucleotide exchange with the oligonucleotide, thereby introducing a unique and selectable SNP in a marker associated with a trait.

Thus, identification of a section of DNA (marker) that is closely linked to the gene associated with the trait of interest which section contains a part that is of a substantially constant nucleotide composition (is low-polymorphic) within a germplasm, determination of the variation at the nucleotide level (A, C, T or G) (or determination of regions of constant nucleotide composition in a marker within a germplasm) in the DNA section, selection of the position in the section that contains a low level, preferably the lowest level of variation at that position , selecting a nucleotide that is different from the nucleotide (including any variation therein) occurring at that position, i.e. that has the lowest occurrence at that position, designing an oligonucleotide that is capable of hybridizing to the sequence adjacent to both sides of the position, and introducing the selected nucleotide (the marker nucleotide) at the position in the DNA of the organism by targeted nucleotide exchange provides for the creation of unique (within a germplasm) selectable markers.

Targeted nucleotide exchange (TNE) is a targeted mutagenesis process in which the mutation is induced by a mismatch base designed in the sequence of an oligonucleotide. TNE has been described in plant, animal and yeast cells. The first TNE reports utilized a so-called chimera that consisted of a self-complementary oligonucleotide that is designed to intercalate at the chromosomal target site. The chimera contains a mismatched nucleotide that forms the template for introducing the mutation at the chromosomal target. In order to select for TNE events, most studies attempt to introduce a single nucleotide change in an endogenous gene that leads to herbicide resistance. The first examples using chimeras came from human cells (see the review Rice et al. Nat. Biotech. 19: 321-326). The use of chimeras has also been successful in the plant species tobacco, rice, and maize (Beetham et al. 1999 Proc. Natl. Acad. Sci. USA 96: 8774-8778; Kochevenko et al. 2003 Plant Phys. 132: 174-184; Okuzaki et al. 2004 Plant Cell Rep. 22: 509-512). Also the TNE activity of single stranded (ss) oligonucleotides has been tested. These have been found to give more reproducible results in wheat, yeast and human cells (Liu et al. 2002 Nuc.Acids Res. 30: 2742-2750; review, Parekh-Olmedo et al. 2005 Gene Therapy 12: 639-646; Dong et al.2006 Plant Cell Rep. 25: 457-65). Targeted mutagenesis (TNE) is also described in a number of patent applications such as WO2005108622, WO2005049795, WO2004033708, WO03075856, WO03027765, WO0210364, WO0192512, WO0187914, WO0173002, WO0114531, WO9515972. TNE has thusfar been described for the purpose of altering genetic expression by knocking in or knocking out genes. The art has not yet provided a workable method for the use of TNE for the introduction of unique selectable markers in a germplasm.

In the first step of the method, a trait of interest is selected. The trait can be a monogenic or multigenic trait or a trait that is governed by a complex of genes, a disease resistance related trait or a yield related trait etc.

In the second step, the locus, or the genetic region that is associated with the trait of interest is selected. Of course, when the trait is multigenic, one of the loci can be selected.

Next, the position of the gene or genetic region on the genetic map is determined. Determination of the genetic map position involves generic methods to which are referred to as linkage analysis or genetic mapping (see for example Griffiths AJF et al. 2005 Introduction to Genetic Analysis, 8th ed. W.H. Freeman and Cie, New York pp. 115 - 137). One of the prerequisites for genetic mapping is a genetic mapping population of two parent lines which differ for the trait of interest and with which a population can be obtained where the trait of interest is segregating in the progeny. Alternatively, in case of a monogenic trait, one could zoom in very effectively at the trait interval by use of bulked segregant analysis BSA(Michelmore et al. 1991 PNAS 88: 9828 - 9832). By applying BSA, there is no need for the construction of a genetic map.

A potential marker sequence is identified within the germplasm (population of the organism that contains genetic variability that can be used for breeding) of interest that is located within a (pre-determined) genetic distance of the gene, i.e. within the vicinity of the trait. Typically, this is within 1 cM of the gene, but this may differ between crops. Such a marker is also indicated as a flanking marker. Genetic mapping and identification of molecular markers in the vicinity of the trait of interest is preferentially executed by multiplex marker technologies. Multiplex marker technologies allow the identification of many markers within a single assay. Various reviews exist on molecular marker technologies (a. o. Rafalski A. 2002 Curr Opin. Plant Biol. 5 94 - 100, Peters J. et al. 2003 TiPS 8 484 - 491, Varshney R. et al. 2005 TiPS 10 621 - 630).

An additional feature which needs to be addressed is the copy number of the underlying marker sequences. Most plant genomes are rich of repetitive sequences (Kumar and Bennetzen JL 1999 Ann Rev Genet 33: 479 - 532) and successful conversion of the initial marker identified into an easy-to-assay marker for routine purpose is relying on low copy DNA sequences. Several methods have been developed to overcome the problem posed by repetitive DNA. Determination of copy number can be achieved by hybridization techniques like Southern blotting. Other methods focus on gene-enrichment strategies like exploiting cDNA sequences, isolation of slowly annealing high Cot DNA (Yuan et al. 2003 Plant J 34: 249 - 255) and methylation filtration (Rabinowicz PD 2003 Meth. Mol Biol 236 21-36). Introduction of a unique marker in the manner as described for this invention also solves this problem of distinguishing between multiple copies of a marker sequence in a genome in a novel an inventive manner and provide a method for the generation of single copy markers in a genome.

Suitable molecular marker technologies are for instance AFLP, RAPDs, SSRs, SFPs and SNPs. Using these technologies, alone or in combination, a large set of markers can be identified that flank the gene of interest.

The set of flanking markers is subjected to a selection process to determine a marker that is located in the vicinity of the trait or at least within the desired distance from the gene (typically expressed in cM). As an alternative, markers that are known, for instance from literature, can be used.

A population of the organism is provided, typically in the form of a breeder's germplasm, or another suitable population comprising a plurality of members that may or may not contain the marker. This population may be pre-screened for the presence of the marker.

From the members of the population that contain the marker, the markers are isolated (for instance cut-out of the electrophoretic gel) and sequenced, i.e. their nucleotide sequence is determined. Determination of DNA sequences of a set of selected markers is executed, for instance, by the Sanger dideoxy-sequencing method but other sequencing methods may suffice.

Thus, after selecting the marker, the marker is also identified in, and isolated from, other members of the same organism to obtain a variety of markers. From each or at least from part of these markers, the sequence is determined. The sequences are aligned and any variation in the DNA makeup of these markers is determined. This means that, at any position of the marker sequence, the occurrence of the respective A, C, T or G is known. The number of members of the population of which markers are sequenced is at least 2, preferably at least 5, more preferably at least 10. In certain embodiments this number may be 25 or 50, depending on the size of the population and the degree wherein polymorphism occurs in the germplasm.

The alignment of the sequences can be performed by hand or by using widely available software tools. The alignment of the sequences provides information of the differences between the markers sequences of different members of the population, i.e. it allows the identification of polymorphism, or lack thereof, in the marker sequence itself. By doing so, information is obtained on regions in the marker sequence that contain less (or none at all) variation in comparison with other regions in the marker. In such a region, one or more nucleotides can be selected that are (relative) constant (are less variable) compared to the other nucleotides in that region. It may even be that there is a region in the marker sequence that is of a constant composition amongst the investigated markers.

To illustrate this, the following scheme is provided:

| Marker X | Sequence |
|---|---|
| Member 1 | XOYQXOXQYXOQYYQYOXQYYQXOYYXOOYX |
| Member 2 | XOYQYOXQYXOQYYQYOXQYYQXOYYXQOYX |
| Member 3 | XOYQYOXQYXOQYYQYOXQYYQXOYYXOOYX |
| Member 4 | XOYQXOXQYXOQYYQYOXQYYQXOYYXQOYX |
| Member 5 | XOYQYOXQYXOQYYQYOXQYYQXOYYXQOYX |
| Member 6 | XOYQYOXQYXOQYYQYOXQYYQXOYYXQOYX |
| Member 7 | XOYQXOXQYXOQYYQYOXQYYQXOYYXQOYX |
| Member 8 | XOYQYOXQYXOQYYQYOXQYYQXOYYXQOYX |

Population Members 1, 3, 4, and 7 contain genetic variation within the marker. This does not have to be the polymorphism that creates the marker itself, but is likely to be just genetic (background) variation. In the sequence there is a region, the 'OXQYXOQYYQYOXQYYQXOYYX' part which does not contain (in the investigated population) any genetic variation, so this region can be depicted as the region of the lowest genetic variation or as the region with the lowest variation in the nucleotides, or as a low-variability region or even a region of constant nucleotide composition within the examined germplasm. This means that, in the schematic example, each of the nucleotides in the constant region, 'OXQYXOQYYQYOXQYYQXOYYX' part qualifies as a position for the introduction of the unique marker nucleotide.

In the following step, an oligonucleotide is designed that is capable of hybridising to the marker sequence at positions adjacent to the potential marker nucleotide position. In the above schematic example this potential marker nucleotide position could be, for instance the second Y (underlined) in XOQYYQYOX. By designing an oligonucleotide that can hybridise to the marker sequence and contains a O at this position (YQOXOOXQY) and, using TNE, introducing the complementary O at the indicated position in the genome now creates a unique marker sequence i.e. OXQYXOQYOQYOXQYYQXOYYX'. This unique DNA sequence (at least within the investigated population) generates a specific and unique marker sequence in the genome of the species. This introduction of a specific marker in a genome can now be used in breeding to select for the desired trait with higher accuracy than the previously known marker.

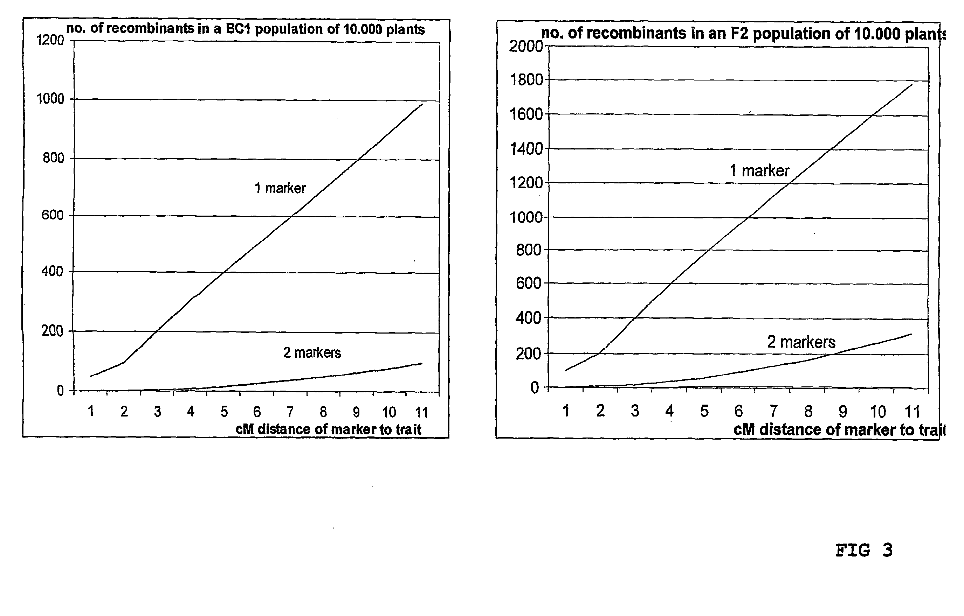

Figure 3, shows the no. of recombinants to be expected in a BC1 and F2 population as a function of the variation in the cM distance of the marker to the trait. In case of a single marker, at least one recombinant (marker present without the trait or vice versa) is expected every 100 or 50 plants respectively. For breeding programs where selections are executed among thousands of plants, this rate is at the outer limit to assure that the diagnostic marker is sufficiently associated with the trait. In current marker development methods, a considerable fine mapping effort is required in order to identify sufficient markers within a 1 cM interval. According to the invention, the markers in the set of selected flanking markers are located at a distance from at most 2 cM, preferably at most 1 cM, more preferably at most 0.5 cM.

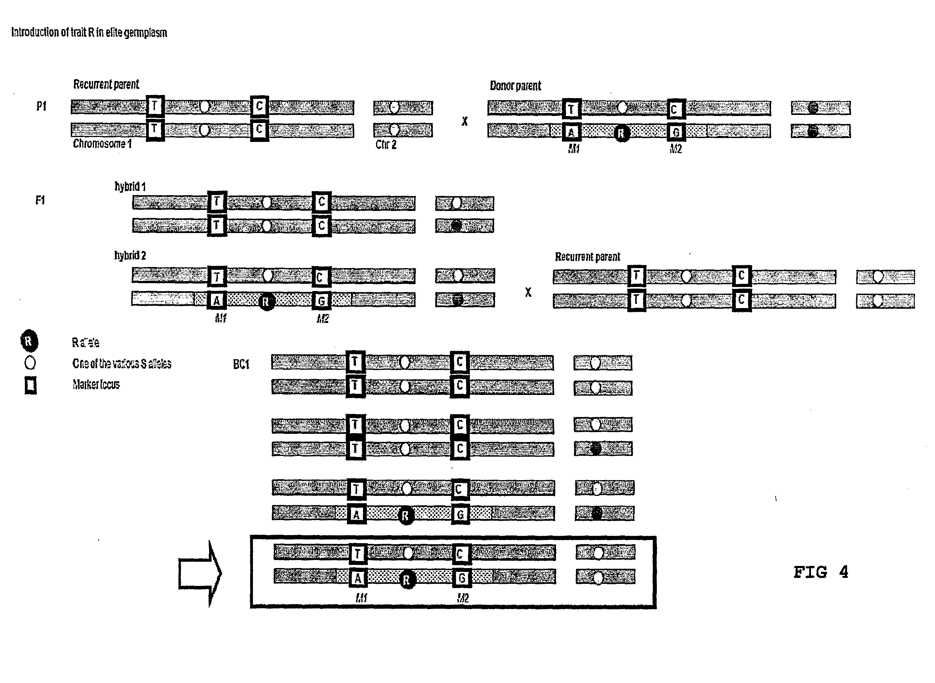

However, according to the invention, in case of the availability of two or more markers, a rate of 1 recombinant per 100 plants can be achieved applying interval sizes of about 10 cM and about 5 cM for BC1 and F2 populations respectively. The use of two of the markers introduced by the method of the invention hence significantly increases the efficiency of the method. As for introgression of a trait in elite germplasm, a backcross strategy is preferred (see Figure 4), the criterion for selecting markers for marker development by TNE is thereby typically set on at least 2 markers within a 10 cM interval of the trait maximally.

In one embodiment where two or more markers are developed, the distances for the (two) selected sets of flanking markers can be significantly higher, as can be seen from Figure 3, preferably at most 10 cM, more preferably at most 5 cM.

The oligonucleotide is introduced in the genome of the organism carrying the locus of interest using targeted mutagenesis (TNE) as outlined herein before. The resulting organism contains a specifically introduced alteration of its genetic sequence that is closely linked to a locus of interest. This specifically introduced alteration can now be used as marker and assayed in any conventional way, whether by molecular marker technologies, PCR-assays, SNP-assays such as SNPWave or otherwise, for instance by sequencing.

Thus, in other words, in the marker sequence, a unique nucleotide is introduced that, as far as can be determined, does not or only very rarely occur in the investigated germplasm of the investigated organism.

The unique marker thus generated can now be assayed using simple assays such as a PCR assay. Determination of DNA sequence variation at the marker loci in the commercially relevant genetic background (germplasm) is achieved by amplifying the marker sequences by PCR on a population such as a germplasm panel by the Sanger dideoxy-sequencing method. This germplasm panel can for instance be representative for the genetic variation at the trait locus in the market segment of the appropriate crop or be representative of the germplasm of a breeder. As an example, for 90 breeding lines, DNA sequences are determined for marker locus *M1*, see also the appended Figures. At position 104 of the DNA sequence either a cytosine (C) or a guanine (G) is observed. Based on this knowledge, an adenine (A) is selected as the nucleotide to be introduced by TNE to create a unique stretch of DNA which is unlikely to exist in the germplasm.

Introduction of the designed sequence variants in the donor plant line by TNE is executed by methods described (Liu et al. 2002 Nuc.Acids Res. 30: 2742-2750; review, Parekh-Olmedo et al. 2005 Gene Therapy 12: 639-646; Dong et al.2006 Plant Cell Rep. 25: 457-65). With TNE, a breeding line is created which has a unique DNA sequence at marker locus *M1*, therefore called *M1**.

Table 1 shows the overall time span of the conventional method of development of a diagnostic marker in plants compared to the novel method described in this invention. Benefits of the novel method are described below, without being intended as limitative.
- The time span of development of a conventional diagnostic marker is rather insecure and may require 12 - 36 months, depending on the trait locus and crop species. In comparison, marker development by means of the present invention takes 13 - 16 months.
- Markers which are artificially introduced by TNE are unique and do not exist in nature. In this way, the introgression segment including the trait and the markers can be the subject of intellectual property protection themselves and their use and application can be monitored in the market.
- For low polymorphic crops like cotton, soybean, cultivated tomato, watermelon and cucumber where it is hard to find any polymorphic marker, markers can be generated by the described method.
- TNE allows the introduction of markers in plant material during the course of the breeding process. It is even possible to create the markers in existing, good performing material.

**Table 1: Comparison of the conventional way diagnostic markers are developed for plant breeding with the novel method described in this invention. The time span is an estimate for each separate step in marker development for a monogenic trait. 1*) Fine mapping by BSA in step 2 of the conventional method is required to identify markers within a 1 cM interval. In crops where the correlation between alleles at different loci is generally low (i.e. the overall extent of linkage disequilibrium is low) only DNA variants with extremely tight linkage to the trait locus are likely to be significantly associated to the trait. In some cases this may require cloning the corresponding gene itself. 2*) Design and construction of a simple PCR assay is dependent on the feasibility in identifying low copy DNA sequences as target for marker conversion. Especially in crops with large genomes like wheat, barley, maize, pepper and lettuce, the identification of low copy sequences for concurrent marker development is not trivial.**

| **Step** | **Conventional Method** | **Time span** | **Novel Method** | **Time span** |
|---|---|---|---|---|
| 1 | Genetic mapping / BSA | 2 months | Genetic mapping / BSA | 2 months |
| 2 | Fine mapping by BSA | 2 - 12 months | Marker identification and copy no. determination | 1 month |
| 3 | Marker identification and copy no. determination | 1 month | Sequencing parent alleles and SNP identification | 0.5 month |
| 4 | Sequencing parent alleles and SNP identification | 0.5 month | Determination sequence variation at marker loci | 0.5 month |
| 5 | Design and construction of simple PCR assay | 6 -12 months | Design of unique marker alleles and TNE at donor line | 9 - 12 months |
| 6 | Test of simple PCR assay on germplasm | 1 month | | |
| **Total** | | 12 - 36 months | | 13 - 16 months |

### Description of the Figures

**Figure 1****:** Schematic representation of marker development at loci *M1* and M2 for a trait to be introgressed from a foreign background. The bars represent a single chromosome.
**Figure 2****:** Schematic representation of the novel sequence variants at loci *M1* and M2 creating markers M1* and M2* used to assay for trait R.
**Figures 3a and 3b.** The number of recombinants to be expected in a BC1 and F2 population as a function of the cM distance of the marker to the trait. The number of recombinants is calculated multiplying the probability of finding a recombinant with the number plants in the population. The probability of finding a recombinant is determined by converting the cM distance into the recombination fraction by use of the Kosambi mapping function multiplied by the chance of the occurrence of a recombinant gamete in the appropriate population type. Kosambi's mapping function is based on empirical data regarding the proportion of double crossovers as the physical distance varies. Kosambi's function adjusts the map distance based on interference which changes the proportion of double crossovers. Kosambi, D.D. 1944. "The estimation of map distances from recombination values." Ann. Eugen. 12:172-75.
**Figure 4****:** Example of BC1 selection for introduction of trait R in an elite background. In this example, a donor parent is used which is heterozygous for trait R. Per plant type, two sets on homologous chromosomes (chromosome 1 = large, chromosome 2 = small) are drawn. The selected BC1 plant contains trait R in heterozygous configuration at chromosome 1 while the composition of chromosome 2 is identical to the composition of the recurrent parent.

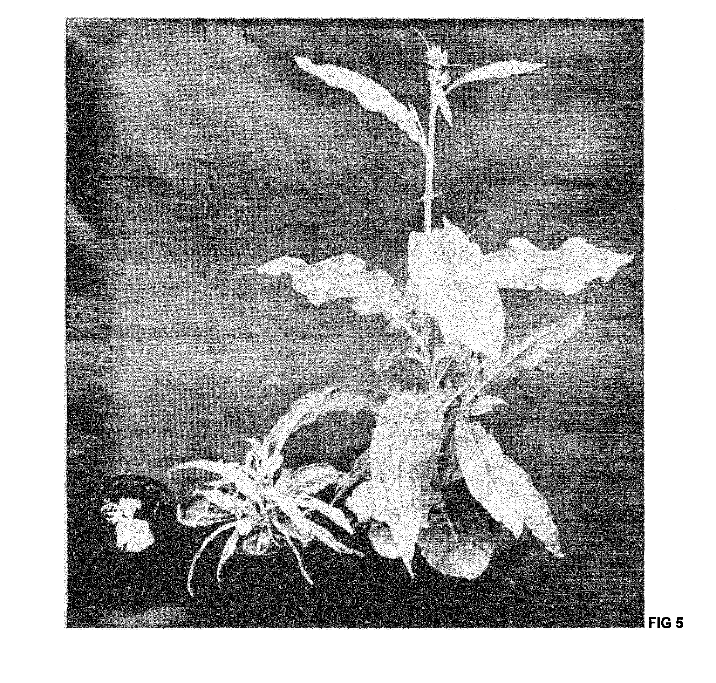

**Figure 5****:** a sensitive, semi-resistant and fully resistant plants of line 25 observed 25 days after chlorsulfuron treatment.

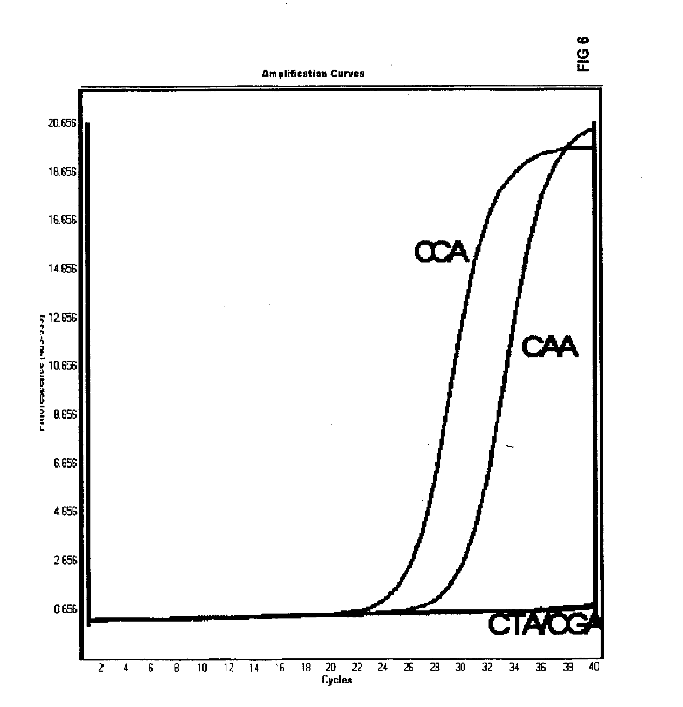

**Figure 6****:** shows amplification curves for wild-type and mutant alleles

### Example

The rationale behind the current invention is to create markers in the vicinity of a trait of interest. These markers are unique in the germplasm and designed based on the sequence knowledge available for the selected stretches of DNA.

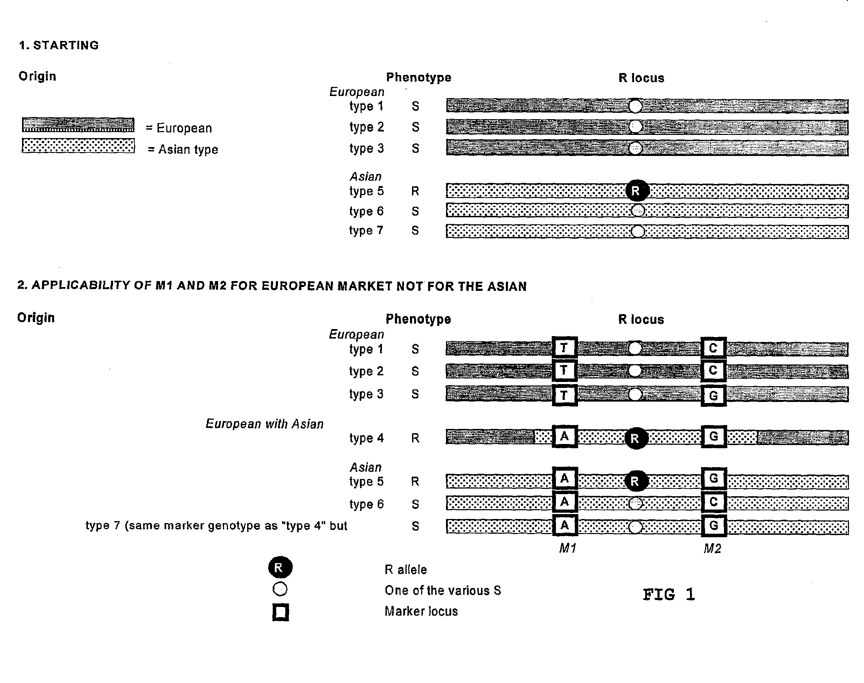

As a first example describing the starting situation, STS markers M1 and M2 have been developed for a disease resistance trait R which is derived from a wild progenitor of a target crop (Figure 1). The disease resistance trait R originates from an Asian accession ("type 5") and has been introgressed in a cultivated European background to create European breeding line "type 4". The STS markers M1 and M2 are developed based on the sequence of marker loci *M1* and M2 which flank the R gene. Only marker M1 has a good predictive value for trait R in the European commercial segment of the target crop. The linkage block containing the Asian resistant haplotype in European background is relatively large.

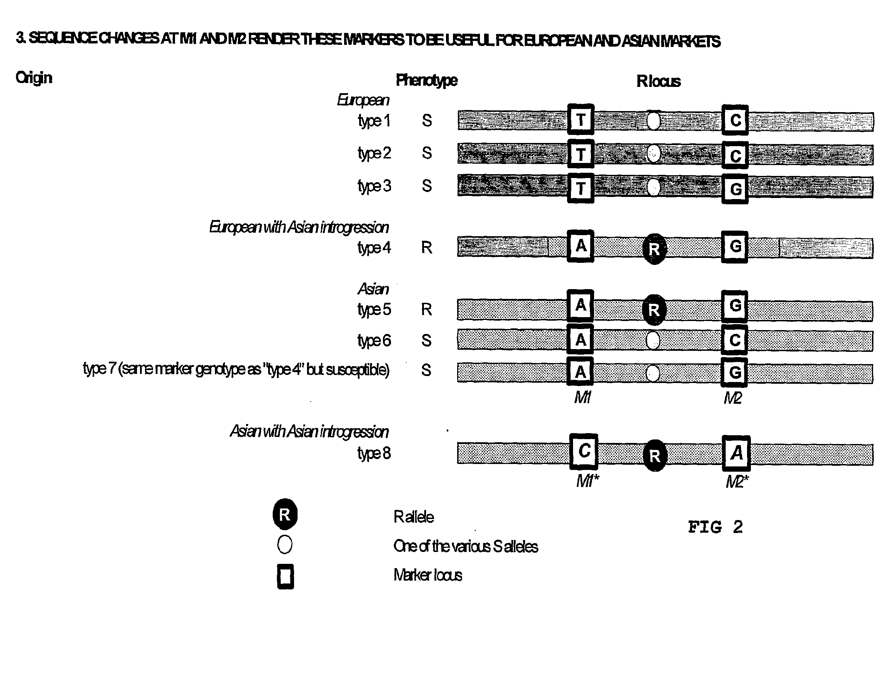

When M1 and M2 are used to test resistant and susceptible material of the target crop in the Asian commercial segment, a poor correlation between the marker and the disease trait is observed. The linkage block containing the resistant haplotype in Asian background is relatively small. For marker development in the starting situation, one could search for markers more closely linked to the gene or, depending on the extent of linkage disequilibrium in the crop, should clone the corresponding gene to assay ultimately the causal polymorphism for trait R. The present invention describes the exploitation of sequence information at marker loci *M1* and M2 instead of re-starting the marker identification and conversion process for R in the Asian germplasm. The knowledge of sequence variation at marker loci *M1* and *M2* can be used to design and generate unique sequence combinations which are perfectly associated with trait R. In Figure 2, the hypothetical situation is drawn where breeding line "type 5" is used to create breeding line "type 8" harboring the novel sequence variants at loci *M1* and M2 which are unique in the germplasm of the target crop in combination with the disease resistance trait R. The newly generated markers M1* and M2* are ideal tools to screen for trait R in both European and Asian germplasm. As they are created artificially, the chance that they occur in nature by accident can be neglected. Hence, markers M1* and M2* are ideal diagnostic markers to screen for trait R among the descendants of the line in which markers M1* and M2* have been introduced.

### Example:

### Creating a pre-defined SNP marker assay for the tobacco genome

### Protoplast isolation

*In vitro* shoot cultures of tobacco *Nicotiana tabacum* cv Petit Havana line SR1 were maintained on MS20 medium with 0.8% Difco agar in high glass jars at 16/8 h photoperiod of 2000 lux at 25°C and 60-70% RH. MS20 medium is basic Murashige and Skoog's medium (Murashige, T. and Skoog, F., Physiologia Plantarum, 15: 473-497, 1962) containing 2% (w/v) sucrose, no added hormones and 0.8% Difco agar. Fully expanded leaves of 3-6 week old shoot cultures were harvested. The leaves were sliced into 1 mm thin strips, which were then transferred to large (100 mm x 100 mm) Petri dishes containing 45 ml MDE basal medium for a preplasmolysis treatment of 30 min. MDE basal medium contained 0.25 g KCI, 1.0 g MgSO₄.7H₂O, 0.136 g of KH₂PO₄, 2.5 g polyvinylpyrrolidone (MW 10,000), 6 mg naphthalene acetic acid and 2 mg 6-benzylaminopurine in a total volume of 900 ml. The osmolality of the solution was adjusted to 600 mOsm.kg⁻¹ with sorbitol, the pH to 5.7. 5 mL of enzyme stock SR1 was then added. The enzyme stock consisted of 750 mg Cellulase Onozuka R10, 500 mg driselase and 250 mg macerozyme R10 per 100 ml, filtered over Whatman paper and filter-sterilized. Leaf tissue digestion was allowed to proceed overnight in the dark at 25°C. The digested leaves were filtered through 50 µm nylon sieves into a sterile beaker. An equal volume of cold KCI wash medium was used to rinse the sieve and was then pooled with the protoplast suspension. KCI wash medium consisted of 2.0 g CaCl₂.2H₂O per liter and a sufficient quantity of KCI to bring the osmolality to 540 mOsm.kg⁻¹. The suspension was transferred to 10 mL tubes and the protoplasts were pelleted for 10 min at 85x g at 4°C. The supernatant was discarded and the protoplast pellets carefully resuspended into 5 mL cold MLm wash medium, which is the macro-nutrients of MS medium (Murashige, T. and Skoog, F., Physiologia Plantarum, 15: 473-497, 1962) at half the normal concentration, 2.2 g of CaCl₂.2H₂O per liter and a quantity of mannitol to bring the osmolality to 540 mOsm.kg⁻¹. The content of 2 tubes was combined and centrifuged for 10 min at 85x *g* at 4°C. The supernatant was discarded and the protoplast pellets carefully resuspended into 5 mL cold MLs wash medium which is MLm medium with mannitol replaced by sucrose.

The content of 2 tubes of protoplasts in MLs wash medium was pooled and 1 mL of KCI wash medium was added above the sucrose solution, with care being taken not to disturb the lower phase. Protoplasts were then centrifuged once again for 10 min at 85x g at 4°C. The interphase between the sucrose and the KCl solutions containing the live protoplasts was carefully collected. An equal volume of KCI wash medium was added and carefully mixed. The protoplast density was measured with a haemocytometer.

### Oligonucleotides

All oligonucleotides were synthesized by Eurogentec (Seraing, Belgium), purified by reverse phase HPLC and resuspended into sterile milliQ water. Prior to use, oligonucleotides were heated up to 95°C for 5 min. Oligonucleotide 06Q262 was designed to introduce a single mismatch (nucleotide underlined) in the tobacco *SurA* gene (accession number X07644) at codon position P194 which would result in a CCA to CAA (P194Q) conversion. Similarly, other oligonucleotides were designed to create a CCA to CTA (P194L) or a CCA to CGA (P194R) conversion (oligonucleotides 06Q263 and 06Q264).
06Q262 5' TCAGTACCTATCATCCTACGTTGCACTTGACCTGTTATAG [SEQ ID 1]
06Q263 5' TCAGTACCTATCATCCTACGTAGCACTTGACCTGTTATAG [SEQ ID 2]
06Q264 5' TCAGTACCTATCATCCTACGTCGCACTTGACCTGTTATAG [SEQ ID 3]

### PEG transformation of protoplasts

The protoplast suspension was centrifuged at 85x g for 10 minutes at 5°C. The supernatant was discarded and the protoplast pellet resuspended to a final concentration of 10⁶.mL⁻¹ in KCI wash medium. In a 10 mL tube, 250 µL of protoplast suspension, 1.6 nmoles of oligonucleotide and 250 µl of PEG solution were gently but thoroughly mixed. After 20 min. incubation at room temperature, 5 mL cold 0.275 M Ca(NO₃)₂ was added dropwise. The protoplast suspension was centrifuged for 10 min at 85x *g* at 4°C. The supernatant was discarded and the protoplast was pellet carefully resuspended in 1.25 mL To culture medium supplemented with 50 µg.mL⁻¹ cefotaxime and 50 µg.mL⁻¹ vancomycin. T₀ culture medium contained (per liter, pH 5.7) 950 mg KNO₃, 825 mg NH₄NO₃, 220 mg CaCl₂.2H₂O, 185 mg MgSO₄.7H₂O, 85 mg KH₂PO₄, 27.85 mg FeSO₄.7H₂O, 37.25 mg Na₂EDTA.2H₂O, the micro-nutrients according to Heller's medium (Heller, R., Ann Sci Nat Bot Biol Veg 14: 1-223, 1953), vitamins according to Morel and Wetmore's medium (Morel, G. and R.H. Wetmore, Amer. J. Bot. 38: 138-40, 1951), 2% (w/v) sucrose, 3 mg naphthalene acetic acid, 1 mg 6-benzylaminopurine and a quantity of mannitol to bring the osmolality to 540 mOsm.kg⁻¹.

The suspension was transferred to a 35 mm Petri dish. An equal volume of To agarose medium was added and gently mixed. Samples were incubated at 25°C in the dark and further cultivated as described below.

### Protoplast cultivation

After 10 days of cultivation, the agarose slab was cut into 6 equal parts and transferred to a Petri dish containing 22.5 mL MAP1AO medium supplemented with 20 nM chlorsulfuron. This medium consisted of (per liter, pH 5.7) 950 mg KNO₃, 825 mg NH₄NO₃, 220 mg CaCl₂.2H₂O, 185 mg MgSO₄.7H₂O, 85 mg KH₂PO₄, 27.85 mg FeSO₄.7H₂O, 37.25 mg Na₂EDTA.2H₂O, the micro-nutrients according to Murashige and Skoog's medium (Murashige, T. and Skoog, F., Physiologia Plantarum, 15: 473-497, 1962) at one tenth of the original concentration, vitamins according to Morel and Wetmore's medium (Morel, G. and R.H. Wetmore, Amer. J. Bot. 38: 138-40, 1951), 6 mg pyruvate, 12 mg each of malic acid, fumaric acid and citric acid, 3% (w/v) sucrose, 6% (w/v) mannitol, 0.03 mg naphthalene acetic acid and 0.1 mg 6-benzylaminopurine. Samples were incubated at 25°C in low light for 6-8 weeks. Growing calli were then transferred to MAP1 medium and allowed to develop for another 2-3 weeks. MAP₁ medium had the same composition as MAP₁AO medium, with however 3% (w/v) mannitol instead of 6%, and 46.2 mg.l⁻¹ histidine (pH 5.7). It was solidified with 0.8% (w/v) Difco agar.

Chlorsulfuron resistant calli were then transferred to RP medium using sterile forceps. RP medium consisted of (per liter, pH 5.7) 273 mg KNO₃, 416 mg Ca(NO₃)₂.4H₂O, 392 mg Mg(NO₃)₂.6H₂O, 57 mg MgSO₄.7H₂O, 233 mg (NH₄)₂SO₄, 271 mg KH₂PO₄, 27.85 mg FeSO₄.7H₂O, 37.25 mg Na₂EDTA.2H₂O, the micro-nutrients according to Murashige and Skoog's medium at one fifth of the published concentration, vitamins according to Morel and Wetmore's medium (Morel, G. and R.H. Wetmore, Amer. J. Bot. 38: 138-40, 1951), 0.05% (w/v) sucrose, 1.8% (w/v) mannitol, 0.25 mg zeatin and 41 nM chlorsulfuron, and was solidified with 0.8% (w/v) Difco agar. After 2-3 weeks, mature shoots > 2cm high and clearly showing leaves and apical meristems were transferred to rooting medium consosting of MS20 medium without any growth regulators.

### PCR amplification of ALS and sequencing

DNA was isolated from chlorsulfuron resistant tobacco calli using the DNeasy kit (Qiagen), and used as a template in a PCR reaction. Conversions of the targeted codons in the tobacco *SurA* gene were detected using the primers 5'GGTCAAGTGCCACGTAGGAT [SEQ ID 4] & 5'GGGTGCTTCACTTTCTGCTC [SEQ ID 5] that amplify a 776 bp fragment of this gene, including codon 194. Nucleotide conversion in the herbicide resistant tobacco callus was confirmed by cloning the PCR products into pCR2.1::TOPO (Invitrogen) and sequencing individual plasmids. Tobacco contains 2 alleles of ALS (*SurA* and *Sur*B). Nucleotide conversion at the P194 codon of either of these loci is sufficient to confer resistance to chlorsulfuron. As tobacco is an allotetraploid species, there are eight possible targets in tobacco at which targeted mutagenesis may have occurred. It is expected that only one out of eight *Sur* alleles had undergone a change to provide the chlorsulfuron resistance. Consequently, seven wild type alleles will also be detected with unchanged sequence. In line with this, it was necessary to sequence >10 plasmid clones containing the PCR product in order to detect one with a codon 194 base conversion. Using this method on chlorsulfuron resistant calli from several tobacco protoplast transfection experiments, it was possible to identify six unique calli, each with a different base change in either *SurA* or *SurB* (Table 1).

**Table 1: Six unique tobacco plants each with a different base conversion at codon 194 of either the SurA or SurB gene.**

| **Plant ID** | **Mutation at P194** | ***sur*A or** |
|---|---|---|
| 02 | CCA → CGA (P194R) | *sur*A |
| 29 | CCA → CGA (P194R) | *sur*B |
| 25 | CCA → CAA (P194Q) | *sur*A |
| 06 | CCA → CAA (P194Q) | *sur*B |
| 15 | CCA → CTA (P194L) | *sur*A |
| 13 | CCA → CTA (P194L) | *sur*B |

### Selfings, seed harvest and Phenotyping

Seeds were obtained from three of the lines shown in Table 1. 96 selfed seeds (M1) from each of these 3 lines were sown in soil and grown in the greenhouse. 36 days after planting, leaf material was harvested from 48 plants per tobacco line for DNA isolation. The 37 day old plants were then also sprayed with a solution of 0.2 % Tween-20, 10 % acetone and 140 µM chlorsulfuron. Chlorsulfuron inhibits the enzyme (acetolactate synthase; ALS) encoded by the *SurA* and *SurB* loci; ALS catalyzes the initial biosynthetic step leading to the synthesis of the amino acids Leu, Ile and Val. The P194 mutations ALS result in an altered form of ALS enzyme which is chlorsulfuron insensitive. All sprayed plants turned somewhat yellow compared to non sprayed plants and some leaves showed a mosaic pattern but this was a previously described temporary phenomenon ("yellow flash"). 20 days after spraying, the plants could be placed into three classes: sensitive, semi-resistant and fully resistant (Figure 1). The sensitive plants showed no growth after spraying and some plants even died. The semi-resistant plants showed reduced growth and the newly formed leaves showed a "shoe-string" like morphology. The fully resistant plants were phenotypically identical to unsprayed control plants. The phenotyping data is shown in Table 2.

**Table 2: Summary of numbers of phenotypes scored of the selfed progeny tobacco lines after chlorsulfuron treatment. Fully resistent plants (F-R), semi-resistent plants (S-R) and sensitive plants (S).**

| **Tobacco line** | **F-R plants** | **S-R plants** | **S plants** | **test ratio** | **χ2 value** | **the deviations are not significant (P≤0.05)** |
|---|---|---|---|---|---|---|
| 25 | 11 | 29 | 8 | 1:2:1 | 2.46 | yes |
| 06 | 13 | 19 | 16 | 1:2:1 | 2.46 | yes |
| 13 | 7 | 29 | 12 | 1:2:1 | 3.13 | yes |

The phenotypic scores of the three tobacco lines fit a Mendelian inheritance (Table 2).

### Development of a qPCR SNP marker assay

Based on the different mutations created in the *SurA* gene, allele-specific sequence polymorphism-derived (AS SPD) primers, featuring a single, allele-selective LNA base at their 3'-end were designed using AlleleID software and purchased from Eurogentec. The LNA (underlined)-containing allele specific antisense primers were:
Sur-581-C: 5'GCATCAGTACCGATCATCCTACGTG 3'detecting the wild type C allele and
Sur-581-G: 5'GCATCAGTACCGATCATCCTACGTC 3',
Sur-581-T: 5'GCATCAGTACCGATCATCCTACGTA 3' and
Sur-581-A: 5'GCATCAGTACCGATCATCCTACGTT 3' detecting the mutated G, T and A alleles, respectively.

The allele specific primers in combination with the forward primer Sur-F: 5' CGCCACCAATCTCGTCAG 3' were used to amplify fragments from genomic DNA of all offspring plants. PCR was performed using the LightCycler 480 real time PCR system in a 384 well PCR block. Reactions contained 5 µl 2 X SYBR green master mix (Roche), 25-50 ng genomic DNA, 5 µM of each primer in a 10 µl PCR reaction. The reaction conditions used were: 95°C for 10 minutes, 18 cycles of 10 sec at 95°C, 20 sec at 66°C

(-0.5 °C every cycle), 20 seconds at 72°C followed by 22 cycles of 10 sec at 95°C, 20 sec at 59°C, 20 seconds at 72°C.

After the amplifications melting curves were produced to check for specificity of the formed products. Ct (cycle treshold) values were then used for analysis.

### Assaying M1 populations with SNP marker assay and correlating with phenotype

The allele specific primers for qPCR were designed based on the *SurA* sequence and differed at one nucleotide (13 nucleotides away from the ALS mutation site) with the *Sur*B sequence. The qPCR results from lines 06 and 13, which contain a mutation in SurB, showed amplification of the wild type alleles (CCA) and the mutated allele(s) (CAA/CTA). Using these *SurA* based primers it was not possible to distinguish between plants either heterozygous and homozygous for the induced mutation in *Sur*B. The qPCR results from line 25 showed amplification of the wild type alleles (CCA) and the mutated allele(s) (CAA) but could also distinguish between heterozygous resistant and homozygous resistant plants. The qPCR results of a homozygous resistant plant showed a lower Ct value in the mutated allele and a higher Ct value in the wild type allele compared to the Ct values of a heterozygous resistant plant.

**Table 3: The scores based on allele specific qPCR data. Homozygous resistant plants (HO-R), heterozygous resistent plants (HE-R) and sensitive plants (S).**

| **Tobacco line** | **HO-R plants** | **HE-R plants** | **R plants** | **S plants** | **test ratio** | **χ2 value** | **the deviations are not significant (P≤ 0.05)** |
|---|---|---|---|---|---|---|---|
| 25 | 11 | 28 | 39 | 9 | 1:2:1 | 1.50 | yes |
| 06 | | | 32 | 16 | 3:1 | 1.78 | yes |
| 13 | | | 35 | 13 | 3:1 | 0.11 | yes |

The allele specific qPCR scores of the three tobacco lines fit with a Mendelian inheritance (Table 3). In addition, there was a clear correlation between the genotype of the plants (homo- or heterozygous for the induced mutation) and the phenotypes of such plants after herbicide treatment, with the semi-resistant plants heterozygous for the mutation and the fully resistant plants homozygous for the mutation.

The phenotypic scoring results per plant were compared with the Q-PCR results and these results correlate for 98 % (Table 4). The very low Chi-square values in Table 4 also show that the phenotypic scores and Q-PCR results correlate. Therefore, the phenotype of an individual can be accurately predicted by marker analysis of a deliberate introduced marker in a unique part of the sequence, and vice versa.

**Table 4: The phenotypic scores compared to the qPCR scores.**

| | **Phenotypic scores** | | | | **Allele specific Q-PCR scores** | | | | | | |
|---|---|---|---|---|---|---|---|---|---|---|---|
| **line** | **HO-R plants** | **HE-R plants** | **R plants** | **S plants** | **HO-R plants** | **HE-R plants** | **R plants** | **S plants** | **test ratio** | **χ2 value** | **the deviations are not significant (P≤ 0.05)** |
| 25 | 11 | 29 | 40 | 8 | 11 | 28 | 39 | 9 | 1:2:1 | 0.16 | yes |
| 6 | 13 | 19 | 32 | 16 | | | 32 | 16 | 3:1 | 0 | yes |
| 13 | 7 | 29 | 36 | 12 | | | 35 | 13 | 3:1 | 0.11 | yes |

In summary, we have shown that a DNA marker can be introduced in a specific plant allele, and that this can be detected using well established SNP detection methods and that the marker will serve to predict/select of the phenotype segregates as expected in the next generation.

## Claims

1. Method for introducing a unique and selectable SNP in a marker associated with a trait in a non-human organism comprising the steps of:
- selecting a trait of interest;
- determining a locus that is associated with the trait;
- determining the genetic map position of the locus;
- identifying a marker that is located within a genetic distance from the locus wherein the genetic distance of the marker is at most 2 cM from the locus for a single marker and at most 10 cM in the case of two or more markers from the locus;
- providing a population of the organism wherein each member of the population contains the marker(s);
- determining the nucleotide sequence of the marker(s) for each of the members of the population;
- aligning the nucleotide sequences of the markers;
- selecting at least one position in the sequence of the markers that contain the same nucleotide in all markers of the population;
- designing an oligonucleotide that is capable of hybridising to the marker sequence adjacent to both sides of the at least one position and wherein the oligonucleotide further contains a nucleotide (the marker nucleotide) at the at least one position that is different from the nucleotide at the at least one position in the marker;
- introduce the marker nucleotide in the DNA of the organism using targeted nucleotide exchange with the oligonucleotide, thereby introducing a unique and selectable SNP in a marker associated with a trait.

2. Method according to claim 1, wherein the marker is identified using molecular marker technologies.

3. Method according to claim 1 or 2, wherein the marker is located at a distance of at most 1 cM, preferably at most 0.1 cM from the locus.

4. Method according to claims 1-2, wherein two or more markers are developed that are located, independently at most 2 cM, preferably at most 0.2 cM from the locus.

5. Method according to claims 1-4, wherein the organism is a plant, animal or micro-organism.

6. Method according to claims 1-5, wherein the organism is a low polymorphic organism, such as cotton (*Gossipyum hirsutum*), soybean (*Glycine max*), cultivated tomato (*Solanum esculentum*), watermelon (*Citrullus lanatus*), cucumber (*Cucumis sativa*)

7. Method according to claim 2, wherein the molecular marker technology is selected from multiplex marker technologies, preferably from the group consisting of AFLP, RAPD, SSR, SFP and/or SNPs.

8. Method according to claim 2, wherein the at least two molecular markers are independently obtained by the multiplex marker technologies from the group consisting of AFLP, RAPD, SSR, SFP and/or SNPs.

9. Method according to claims 1-8, wherein the DNA of the organism is from a donor line suitable for breeding.

10. Method according to any of the previous claims for the introduction of a unique and selectable marker in a multi-copy DNA segment

11. Use of the method of claims 1-10 for creating one or more artificial markers in existing breeding material.

12. Use of the method of claims 1-10 for introducing one or more markers in genetically modified material.

## Patentansprüche

1. Verfahren zur Einführung eines einzigartigen und selektierbaren SNP in einen Marker, der mit einem Merkmal in einem nicht-menschlichen Organismus assoziiert ist, umfassend die Schritte:
- Auswählen eines Merkmals von Interesse;
- Bestimmen eines Lokus, der mit dem Merkmal assoziiert ist;
- Bestimmen der genetischen Kartenposition des Lokus;
- Identifizieren eines Markers, der sich in einer genetischen Entfernung von dem Lokus befindet, wobei die genetische Entfernung des Markers höchstens 2 cM von dem Lokus für einen einzelnen Marker und höchstens 10 cM im Fall von zwei oder mehr Markern von dem Lokus aus ist;
- Bereitstellen einer Population des Organismus, wobei jedes Mitglied der Population den Marker (die Marker) enthält;
- Bestimmen der Nucleotidsequenz des Markers (der Marker) für jedes der Mitglieder der Population;
- Ausrichten der Nucleotidsequenzen der Marker;
- Auswählen wenigstens einer Position in der Sequenz der Marker, die dasselbe Nucleotid in allen Markern der Population enthält;
- Entwickeln eines Oligonucleotids, das fähig ist, an die Markersequenz, benachbart zu beiden Seiten der wenigstens einen Position, zu hybridisieren und wobei das Oligonucleotid außerdem ein Nucleotid (das Markernucleotid) an der wenigstens einen Position enthält, das von dem Nucleotid an der wenigstens einen Position in dem Marker unterschiedlich ist;
- Einführen des Markernucleotids in die DNA des Organismus unter Verwendung eines targetierten Nucleotidaustausches mit dem Oligonucleotid, wodurch ein einzigartiger und selektierbarer SNP in einen Marker, der mit dem Merkmal assoziiert ist, eingeführt wird.

2. Verfahren gemäß Anspruch 1, wobei der Marker unter Verwendung molekularer Markertechnologien identifiziert wird.

3. Verfahren gemäß Anspruch 1 oder 2, wobei der Marker in einer Entfernung von höchstens 1 cM, vorzugsweise höchstens 0,1 cM, von dem Lokus lokalisiert ist.

4. Verfahren gemäß Anspruch 1 bis 2, wobei zwei oder mehr Marker entwickelt werden, die unabhängig höchstens 2 cM, vorzugsweise höchstens 0,2 cM, von dem Lokus lokalisiert sind-

5. Verfahren gemäß den Ansprüchen 1 bis 4, wobei der Organismus eine Pflanze, ein Tier oder ein Mikroorganismus ist.

6. Verfahren gemäß den Ansprüchen 1 bis 5, wobei der Organismus ein wenig polymorpher Organismus ist, z.B. Baumwolle (*Gossipyum hirsutum),* Sojabohne (*Glycine max*), kultivierte Tomate (*Solanum esculentum),* Wassermelone (*Citrullus lanatus*), Gurke (*Cucumis sativa*).

7. Verfahren gemäß Anspruch 2, wobei die molekulare Marker-Technologie aus Multiplex-Marker-Technologien ausgewählt ist, vorzugsweise aus der Gruppe, bestehend aus AFLP, RAPD, SSR, SFP und/oder SNPs.

8. Verfahren gemäß Anspruch 2, wobei die wenigstens zwei molekularen Marker unabhängig durch die Multiplex-Marker-Technologien aus der Gruppe, bestehend aus AFLP, RAPD, SSR, SFP und/oder SNPs, erhalten werden.

9. Verfahren gemäß Ansprüchen 1 bis 8, wobei die DNA des Organismus aus einer Donorlinie ist, die zur Züchtung geeignet ist.

10. Verfahren gemäß einem der vorangehenden Ansprüche für die Einführung eines einzigartigen und selektierbaren Markers in ein Multi-Kopien-DNA-Segment.

11. Verwendung des Verfahrens gemäß Ansprüchen 1 bis 10 zur Erzeugung eines oder mehrerer künstlicher Marker in existierendem Züchtungsmaterial.

12. Verwendung des Verfahrens gemäß Ansprüchen 1 bis 10 zur Einführung eines Markers oder mehrerer Marker in genetisch modifiziertes Material.

## Revendications

1. Procédé d'introduction d'un polymorphisme de nucléotide unique (SNP) dans un marqueur associé à un caractère dans un organisme non humain comprenant les étapes de :
- sélection d'un caractère d'intérêt ;
- détermination d'un locus qui est associé au caractère ;
- détermination de la position du locus dans la carte génétique ;
- identification d'un marqueur qui est situé à une distance génétique du locus, la distance génétique du marqueur étant d'au plus 2 cM du locus pour un marqueur unique et d'au plus 10 cM, dans le cas de deux marqueurs ou plus, du locus ;
- fourniture d'une population de l'organisme, chaque membre de la population contenant le(s) marqueur(s) ;
- détermination de la séquence nucléotidique du ou des marqueur(s) pour chacun des membres de la population ;
- alignement des séquences nucléotidiques des marqueurs ;
- sélection d'au moins une position dans la séquence des marqueurs qui contient le même nucléotide dans tous les marqueurs de la population ;
- conception d'un oligonucléotide qui est capable de s'hybrider à la séquence de marqueur adjacente aux deux côtés de l'au moins une position et l'oligonucléotide contenant en outre un nucléotide (le nucléotide marqueur) à l'au moins une position qui est différent du nucléotide à l'au moins une position dans le marqueur ;
- introduction du nucléotide marqueur dans l'ADN de l'organisme au moyen d'un échange de nucléotide ciblé avec l'oligonucléotide, de manière à introduire un SNP unique et sélectionnable dans un marqueur associé à un caractère.

2. Procédé selon la revendication 1, dans lequel le marqueur est identifié en utilisant des technologies de marqueur moléculaire.

3. Procédé selon la revendication 1 ou 2, dans lequel le marqueur est situé à une distance d'au plus 1 cM, de préférence d'au plus 0,1 cM du locus.

4. Procédé selon les revendications 1 à 2, dans lequel deux marqueurs ou plus sont développés qui sont situées, indépendamment, à au plus 2 cM, de préférence au plus 0,2 cM du locus.

5. Procédé selon les revendications 1 à 4, dans lequel l'organisme est une plante, un animal ou un micro-organisme.

6. Procédé selon les revendications 1 à 5, dans lequel l'organisme est un organisme faiblement polymorphe, tels que le coton (*Gossipyum hirsutum*), le soja (*Glycine max*), la tomate cultivée (*Solanum esculentum*), la pastèque (*Citrullus lanatus*), le concombre (*Cucumis sativa*).

7. Procédé selon la revendication 2, dans lequel la technologie de marqueur moléculaire est choisie parmi des technologies de marqueur multiplexes, de préférence dans le groupe constitué de AFLP, RAPD, SSR, SFP et/ou SNP.

8. Procédé selon la revendication 2, dans lequel les au moins deux marqueurs moléculaire sont indépendamment obtenues par les technologies de marqueur multiplexes dans le groupe constitué de AFLP, RAPD, SSR, SFP et/ou SNP.

9. Procédé selon les revendications 1 à 8, dans lequel l'ADN de l'organisme est d'une lignée donneuse adaptée pour la reproduction.

10. Procédé selon l'une quelconque des revendications précédentes pour l'introduction d'un marqueur unique et sélectionnable dans un segment d'ADN multi-copies.

11. Utilisation du procédé des revendications 1 à 10 pour créer un ou plusieurs marqueurs artificiels dans un matériel de reproduction existant.

12. Utilisation du procédé des revendications 1 à 10 pour introduire un ou plusieurs marqueurs dans un matériel génétiquement modifié.
